# EUROPEAN PATENT SPECIFICATION

(11) **EP 2 170 369 B1**
(45) Date of publication and mention of the grant of the patent: **23.05.2012**
(21) Application number: 08773368.9
(22) Date of filing: 06.06.2008
(51) Int. Cl.: A61K 38/19, A61K 38/21, C12N 5/0793, A61P 31/22

(54) **VIRAL LATENCY MODEL**
VIRUSLATENZMODELL
MODÈLE DE LATENCE VIRALE

(30) Priority: 08.06.2007 EP 07011225
(43) Date of publication of application: 07.04.2010
(73) Proprietor: Universiteit Gent, 9000 Gent (BE)
(72) Inventor: DE REGGE, Nick, B-9890 Gavere (BE); FAVOREEL, Herman, B-9820 Merelbeke (BE); NAUWYNCK, Hans, B-9930 Zomergem (BE)
(74) Representative: Laenen, Bart Roger Albert
(86) International application number: PCT/EP2008/004530
(87) International publication number: WO 2008/148563

(56) References cited:
- EP-A- 0 535 390
- WO-A-00/65355
- WO-A-91/11718
- WO-A-99/16891
- US-A1- 2003 032 006
- DECMAN VILMA ET AL: "Gamma interferon can block herpes simplex virus type 1 reactivation from latency, even in the presence of late gene expression" JOURNAL OF VIROLOGY, vol. 79, no. 16, August 2005 (2005-08), pages 10339-10347, XP002458814 ISSN: 0022-538X

## Description

### Field of the Invention

The invention relates generally to the field of virology. More particularly, the present invention relates to *in vitro* models for viral latency. In particular to latently infected cultures of continuous and primary cell lines that are permissive for said viruses, and to the use thereof in methods to identify anti-viral compounds. More in particular to identify compounds which are either able to modulate the induction of viral latency in the aforementioned cell cultures, or which are able to retain the viruses in the aforementioned cells in their latent form.

### Background to the Invention

Alphaherpesviruses are a subfamily of the herpesviruses containing closely related human and animal pathogens. Herpes simplex virus 1 (HSV-1) is the prototype of human alphaherpesviruses causing diseases ranging from mild labialis and stomatitis to blinding keratitis and in some rare cases to lethal encephalitis. Important animal alphaherpesviruses include the porcine pseudorabies virus (PRV) and bovine herpesvirus 1 (BoHV-1) causing respiratory symptoms, abortions and/or neurological symptoms.

Primary infection of the host starts with a productive infection in epithelial cells of the upper respiratory tract, followed by virus entry in axons that innervate the infected mucosal surface. Then, the virus is transported retrogradely to sensory neuronal cell bodies in ganglia of the peripheral nervous system, with neurons of the trigeminal ganglion being the predominant target cells for HSV-1, PRV and BoHV-1. Mostly, a brief period of replication in the neurons is followed by the establishment of a latent infection in which functional viral genomes are retained in neuronal nuclei without virus production, causing a lifelong infection of the host.

Stressful stimuli such as immunosuppression, trauma and heat, lead to periodic reactivation from this latent state, which may result in new virus production and recurrent disease after anterograde axonal transport to the site of primary infection. Reactivated HSV viruses are responsible for causing recurrent epithelial infections that can occur in up to 89% (US2003/032006) of infected individuals.

Although latency obviously is a critical aspect of herpesviruses lifecycle, many of the mechanisms resulting in establishment, maintenance and reactivation from latency are not well understood. This is partly due to the fact that a universally accepted in vitro model that supports herpesvirus latency is missing.

Where animal models reproduce certain aspects of HSV latency in humans, a number of limitations in these models make interpretation of reactivation data challenging. Animal models limitations include: (i) latency and reactivation events that are influenced by viral strains with different primary growth phenotypes, (ii) the limited number of neurons latently infected in animal models, and (iii) inaccurate quantitation of reactivation events when measuring virus production at the recurrent site as a result of influences of transport, replication in epithelium, and the immune response.

In an attempt to overcome the limitations of animal models, ex vivo explant models of latently HSV infected murine ganglia and in vitro cell culture models of HSV latency have been developed. A major advantage of explant models and in vitro cell culture models includes the ability to observe virus at the single cell level without the overlay of immunological and other events that modulate the eventual appearance of virus in the host. Nevertheless, ex vivo explant models have their drawbacks, preparation of dissected ganglia is inconvenient, material is limited, animal use is required, and axotomy introduces traumatic factors that influence reactivation of virus. In addition, the number of latently infected neurons per ganglion is variable and/or low.

Accordingly, development of cell culture models with neuronal characteristics that lack the restrictive requirements of ex vivo explant models would be advantageous for understanding the molecular mechanisms of the establishment, maintenance and reactivation stages of HSV latency.

Over the past 25 years, cell culture systems have been published in which herpesviruses can be directed into a 'latency-like' state using either exogenous antiviral compounds, such as acyclovir, zidovudine (AZT), lamivudine (3TC), indinavir (IDV), ganciclovir, or similar compounds and analogs thereof; attenuated mutant viruses; or non-permissive cells or non-permissive virus growth conditions (e.g. temperature). A first series of studies describes the use of specific exogenous antiviral compounds, i.e. guanosine analogs, to induce a 'latency-like' state of viral infection in different kinds of cell cultures. These compounds are known antivirals that stop the virus reproduction cycle at the time of DNA replication. This is not a natural way of latency induction and the resulting virus infections status is therefore generally not referred to as latency but as 'quiescent infection'. This approach includes the risk that the virus is present in this artificial quiescent infected cells in a conformation that is different than when a natural latent state is induced. Hence, products that prevent reactivation from this artificial quiescent state may not have a similar effect on reactivation from true, bona fide alphaherpesvirus latency.

Other studies describe the use of other exogenous (as in non-naturally occurring) antiviral compounds such as 1-b-arabinosyl cytosine (mitosis inhibitor) or cycloheximide (protein synthesis inhibitor); elevated temperature, replication-impaired genetically altered virus strains; and/or non-permissive cell lines as mechanisms to bring HSV in a quiescent state of infection. Again, these conditions artificially block replication of the virus rather than establishing latency and are not physiologically relevant models for herpesvirus latency. A representative example of the above-mentioned cell culture models is US application US2003/0032006 or US patent US 6,573,041 (Miller et al.) that provides the use of a PC12 cell line 'quiscently' infected with HSV-2. In this case the host cell is non-permissive for HSV-2 viral infection, i.e. does not support replication of the virus, and the 'quiscent state' can be seen as a restricted permissiveness of this host cell to the presence of virus particles, but does not represent a state of true natural herpesvirus latency.

It is accordingly an object of the present invention to provide a fysiological relevant *in vitro* cell culture model of herpesvirus latency that does not artificially impede virus replication, and closely mimics natural viral latency in a cell.

### Brief Description of the Drawings

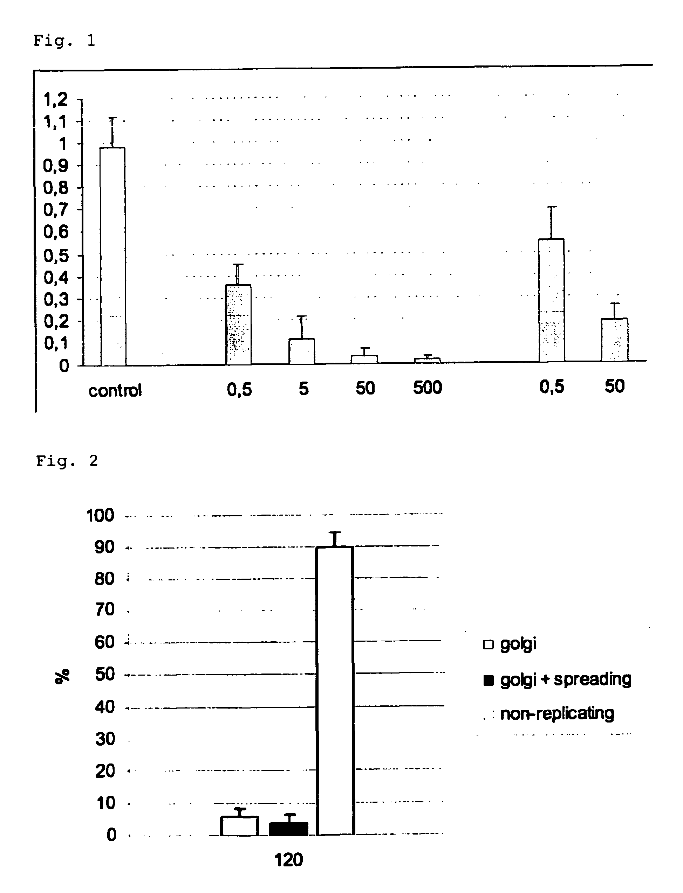

- **Figure 1**: Provides the ratio of the number of PRV late antigen positive neurons in the inner chamber to the number of axons in the outer chamber in the two-chamber model upon treatment with IFN-α (0,5 to 500 U/ml - left panel) and IFN-γ (0,5 to 50 U/ml) at 24 hpi. Data shown represent means ± SEM triplicate assays.
- **Figure 2**: Provides the percentage of PRV infected porcine TG neurons in the two chamber model that show; (i) no detectable expression of late viral antigens (white bars); (ii) late viral antigen expression limited to the golgi (grey bars); and (iii) spread of the infection to neighboring cells (black bars); as determined using using polyclonal porcine FITC-labeled anti-PRV antibody upon treatment with 500 U/ml of IFN-α up till 120 hpi. Data shown represent means ± SEM triplicate assays.

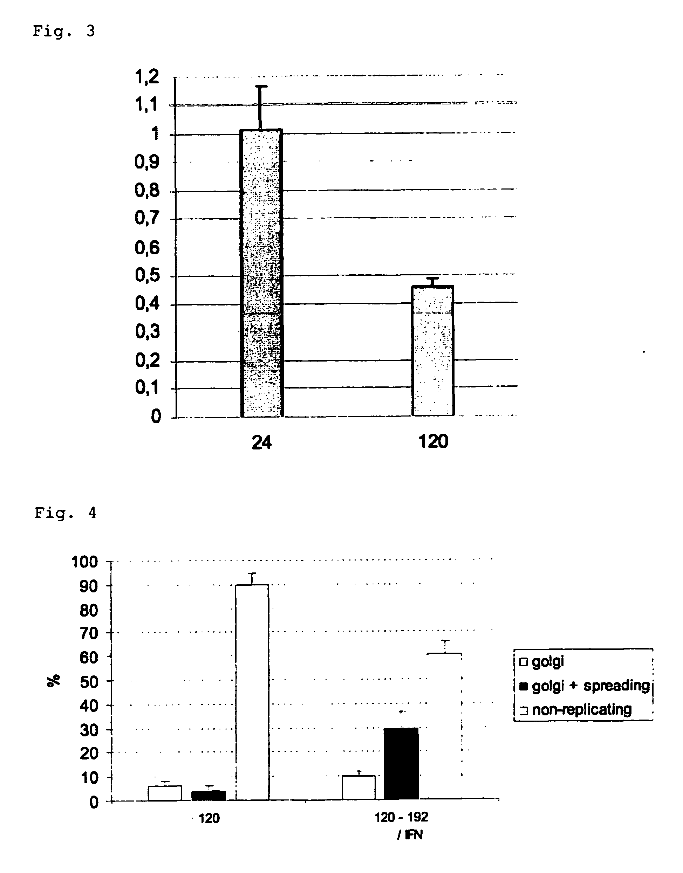

- Figure 3: Provides the ratio of the number of IE180 positive neurons in the inner chamber to the number of axons in the outer chamber in the two-chamber model upon treatment with 500 U/ml of IFN-α at 24 hpi and 120hpi respectively. Data shown represent means ± SEM triplicate assays.
- **Figure 4**: Provides the percentage of PRV infected porcine TG neurons in the two chamber model that show; (i) no detectable expression of late viral antigens (white bars); (ii) late viral antigen expression limited to the golgi (grey bars); and (iii) spread of the infection to neighboring cells (black bars); as determined using polyclonal porcine FITC-labeled anti-PRV antibody upon treatment with 500 U/ml of IFN-α up till 120 hpi (left panel) and at 192 hpi wherein the cells where cultured from 120-192 hpi in the absence of IFN-α (right panel). Data shown represent means ± SEM triplicate assays.

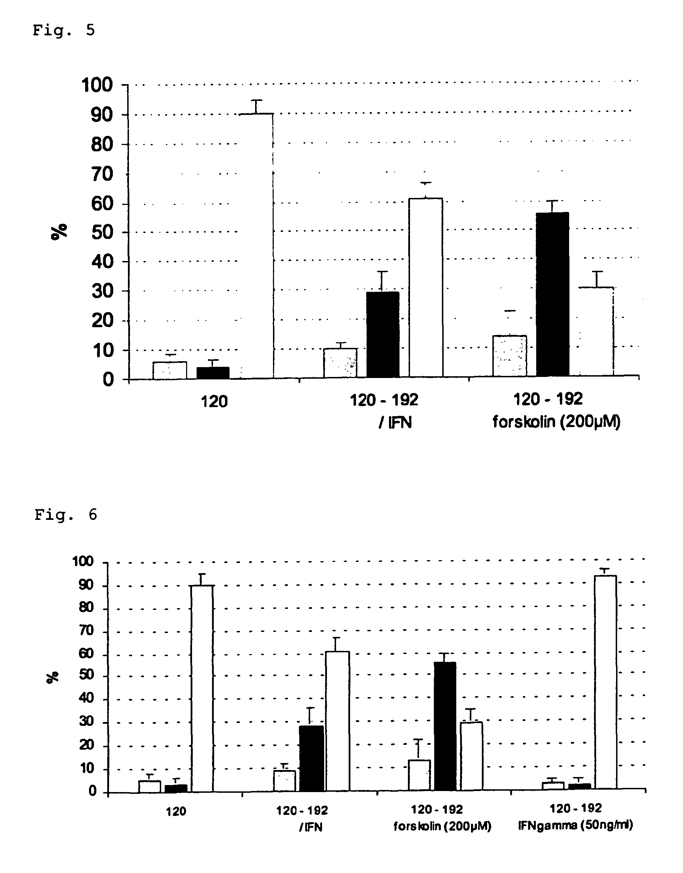

- Figure 5: Is the same as figure 4 above but now complemented with the effect of reactivation with forskolin for 3 days without the suppressing effect of IFN-α. Data shown represent means ± SEM triplicate assays.
- Figure 6: Is the same as figure 5 above but now complemented with the effect of treatment with IFN-γ for 3 days without the suppressing effect of IFN-α. Data shown represent means ± SEM triplicate assays.

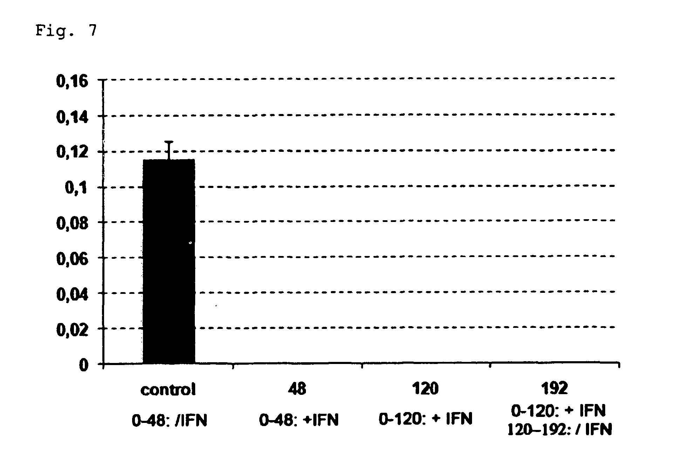

- **Figure 7**: Provides the ratio of the number of HSV-1 gD antigen positive neurons in the inner chamber to the number of axons in the outer chamber for i) control, non-treated infection experiments at 48hpi and ii) IFN-α (500 U/ml) treated two-chamber models at 48 and 120hpi and iii) IFN-α (500 U/ml) treated two-chamber models at 192hpi wherein the cells where cultured from 120-192 hpi in the absence of IFN-α. Data shown represent means ± SEM triplicate assays.

### Summary of the Invention

This invention relates to in vitro methods to induce viral latency in a cell, and is based on the finding that endogenous (as in naturally occurring) inflammatory cytokines,

in particular interferon alpha, bring about a reactivatable latent state of viral infection in alphaherpesvirus infected cells.

As already mentioned hereinbefore, compared to the methods currently known in the art, the methods of the present invention differ in that said reactivatable latent state can be achieved in virus 'permissive' cells, i.e. cells in which said virus can complete its replication cycle, under physiologically relevant culture conditions, without the use of attenuated mutant viruses and/or the presence of exogenous (as in non-naturally occurring) antiviral compounds.

As already mentioned hereinbefore, the methods as provided in the different embodiments of the present invention are performed under physiologically relevant conditions. Said conditions are meant to refer to the normal (wild-type) physiologically conditions known for the viral strain and host used. Such as for example at an incubation temperature of about and between 34- 40°C, and using the art known and established cultivation conditions for the host cells used. In general, the reactivatable latent state can be achieved without the need of an elevated incubation temperature, and/or the use of attenuated viruses, and/or the use of exogenous antiviral compounds. In a particular embodiment of the present invention, the cells (permissive cells) are neuronal cells infected with herpesviridae. More in particular in neuronal cells infected with herpes simplex virus 1, herpes simplex virus 2 and porcine alphaherpesvirus, i.e. pseudorabies virus (PRV). In a further embodiment the cells are primary cultures of porcine trigeminal ganglion infected with porcine alphaherpesvirus, i.e. pseudorabies virus (PRV) or herpes simplex virus 1.

It is accordingly a first objective of the present invention to provide a method to induce viral latency in an alphaherpesvirus infected neuron-like cell(s) said method comprising contacting said cell(s) with interferon alpha and characterized in that permissive cells and no attenuated virus mutants or exogenous antiviral compounds are used.

In the methods of the present invention the cells are contacted with the cytokine till the cells are free of detectable viral proteins, in particular of detectable early viral proteins, more in particular of detectable immediate early viral proteins. The presence of viral proteins in cells is typically determined using viral specific antibodies, in particular using polyclonal anti-viral serum, more in particular using early viral protein-specific antibodies, more in particular using immediate early viral protein-specific antibodies. Other methods to determine the presence of viral proteins in the media are known to the person skilled in the art and include but are not limited to immunohistochemistry, Western blotting, in situ hybridisation, and (RT-)PCR, titrations.

In a particular embodiment, the viral latently infected cells obtainable using the methods of the present invention, consists of a primary culture of porcine trigeminal neurons infected with herpes simplex virus 1 (HSV-1) or porcine alphaherpesvirus, i.e. pseudorabies virus (PRV) (PRV), obtained by;
- treating a primary culture of porcine trigeminal neurons with interferon alpha prior to the infection of said cells with PRV or HSV-1, and
- contacting said PRV or HSV-1 infected cells with interferon alpha till at least 40% of the infected cells no longer show detectable viral protein expression, in particular early viral protein expression, more in particular immediate viral protein expression.

The viral protein expression can be determined using a variety of protein measurement techniques, including the use of viral specific antibodies; in particular using polyclonal anti-viral serum, such as for example a polyclonal anti-PRV serum when the cells are infected with PRV.

In a second objective, the present invention provides the use of the aforementioned methods and/or cells in methods to identify compounds capable to modulate the induction of viral latency in a neuron-like cell or the reactivation of virus from latently infected cells.

In a first aspect said methods comprise;
- applying the methods according to the invention in the presence and absence of the compound to be tested; and
- compare the level of viral latency obtained in the presence and absence of the compound to be tested;
wherein a compound capable to change the level of viral latency when compared to the level obtained in the absence of said compound, is a compound capable of modulating the induction of viral latency in a cell. In one embodiment of the present invention, the level of viral latency is determined by assessing (i) the percentage of cells with no detectable expression of viral proteins, in particular no detectable expression of early viral proteins, more in particular no detectable expression of immediate early viral proteins and (ii) assessing within the percentage of cells of step (i), the percentage of cells that show reactivation upon treatment with specific stimuli. Reactivation is defined as the detectable expression of viral proteins in cells, in particular the production of infectious virus in cells. Reactivation stimuli are known to the person skilled in the art and include but are not limited to forskolin, UV irradiation, heat shock, and corticosteroid treatment.

A compound capable to increase the percentage of cells in which no viral proteins can be detected upon cytokine treatment and wherein said cells retain the capability of viral reactivation, when compared to the percentages obtained in the absence of the compound to be tested, is identified as a compound that promotes viral latency in a cell.

In a second aspect, said methods comprises contacting a viral latently infected cell obtainable using the methods of the present invention with a compound to be tested and determine whether said compound is capable to modulate, in particular prevent viral reactivation of said latently infected cells, wherein reactivation is determined as described hereinbefore. Compounds thus identified can be used in the treatment of latent viral infections.

### Detailed description of the Invention

### Definitions

"virus" means the definition as understood by those skilled in the art, as well as viroid particles such as prions, and including natural and artificial alterations thereof (e.g. mutations, such as temperature sensitive mutations, including deletions insertions, etc.).

"Attenuated virus mutants" refers to a weakened, less vigorous virus that is still capable of stimulating an immune response and creating immunity. Such an attenuated virus can be the result of natural or artificial alterations of the wild type virus.

"Compounds" as used includes, but is not limited to; small molecules including both organic and inorganic molecules with a molecular weight of less than 2000 daltons; proteins; peptides; antisense oligonucleotides; siRNAs; antibodies, including both monoclonal and polyclonal antibodies; ribozymes; etc.

In this respect "endogenous antiviral compounds" are compounds that are naturally occurring in a mammal, whether or not as a result of a virus infection, and that directly or indirectly interfere with optimal viral growth or replication in a cell, e.g. cytokines and virus specific antibodies. "Exogenous antiviral compounds" are compounds that are not naturally occurring in a mammal, whether or not as a result of a virus infection, that can interfere with optimal viral growth or replication in a cell such as viral replication inhibitors, for example the guanosine analogues acyclovir, penciclovir, and (E)-5-(2-bromobinyl)-2'-deoxyuridine (BVDU), valacyclovir and cydofovir; other replication inhibitors such as phosphono acetic acid; mitosis inhibitors such as 1-b-arabinosyl cytosine; protein synthesis inhibitors such as for example cycloheximide.

Cells that are cultured directly from an animal or person are known as "*primary cells*". With the exception of some derived from tumours, most primary cell cultures have limited lifespan. After a certain number of population doublings cells undergo the process of senescence and stop dividing, while generally retaining viability. As used in the methods of the present invention, the "primary cells" are derived from; sensory neurons, such as for example from trigeminal neurons or dorsal root ganglion neurons; sympathetic neurons, such as for example superior cervical ganglion neurons and from leukocytes such as for example B-lymphocytes, T-lymphocytes, dendritic cells or monocytes. In particular embodiments of the present invention, the "primary cells" are derived from trigeminal neurons, more in particular from porcine trigeminal neurons.

This in contrast to "continuous cells" also known as "an established" or "immortalized" cell line that has acquired the ability to proliferate indefinitely either through random mutation or deliberate modification, such as artificial expression of the telomerase gene. There are numerous well established cell lines representative of particular cell types. In the context of the present invention, the continuous cells are selected from the group consisting of neuron-like cells such as for example a continuous cell line derived from trigeminal ganglion neurons, PC12 cells (CRL-1721)or ND7 cells; and non-neuronal cells such as for example swine testicle cells, swine kidney cells (e.g. PK15 (CCL-33), SK-RST(CRL-2842)), epithelial cell cultures, skin keratinocytes (e.g. HEK001 (CRL-2404), CCD1102 (CRL-2310)), Vero cells (CCL-81), human fetal lung fibroblasts (e.g. HFL1 (CCL-153)), human embryonic lung cells (e.g. HEL299 (CCL-137))or lymphocyte cell cultures.

As used herein, the terms "permissiveness of a cell(s)", "permissivity of cell(s)" and "permissive cell(s)" refers to the ability in which a particular virus, i.e. an Alphaherpesvirus, such as for example herpes simplex virus 1, herpes simplex virus 2, or porcine alphaherpesvirus, can complete its replication cycle in a given cell in normal (standard) culture conditions and without the addition of reactivating stimuli. This, in contrast to "non-permissive" cells that do not support complete replication of a virus under such standard culture conditions and without the addition of reactivating stimuli.

As is known by the skilled artisan, viral replication consists of two stages.

A first stage, also referred to as "early stage" in which genes that encode for enzymes and regulatory proteins needed to start viral replication are transcribed. Without the "immediate early proteins", viral replication does not initiate. For example, pseudorabies virus, a.k.a. porcine alphaherpesvirus, expresses IE180 (the orthologue of ICP4 of HSV).

A second stage, also referred to as "late stage" in which genes that encode structural proteins, i.e. proteins needed for assembly of the mature virus are transcribed. "late viral proteins" expressed at this stage of the viral replication cycle include small and large viral capsid proteins, assembly proteins and envelope proteins. For example, in herpesviruses "late viral proteins" include but are not limited to glycoprotein C, glycoprotein H, and major capsid protein VP5.

As already mentioned hereinbefore, it is an object of the present invention to provide a method to induce viral latency in alphaherpesvirus infected neuron-like cells. The stage of "viral latency" as used herein refers to the stable (e.g. in an environment without antiviral pressure) presence of viral genomes in the nuclei of said cells without virus production that can subsequently be reactivated to resume production of viral proteins.

An "interferon alpha receptor agonist" as used herein refers to compounds that are capable to activate the interferon alpha receptor in analogy with binding of the natural ligand, i.e. interferon alpha and includes interferon alpha analogs such as for example interferons, synthetic interferons, pegylated interferons, fusion proteins comprising an interferon and a heterologous protein, shuffled interferons; antibody specific for an interferon receptor; non-peptide chemical agonists; and the like.

### In vitro model

As already mentioned hereinbefore; it is a first objective of the present invention to provide a method to induce viral latency in a alphaherpesvirus infected neuron-like cell(s) said method comprising contacting said cell(s) with interferon alpha and characterized in that permissive cells are used and that no attenuated virus mutants, and/or no exogenous (as in non-naturally occurring) antiviral compounds are used. The cells used in the methods of the invention are primary or continuous cells as defined above.

In particular embodiments of the present invention the primary cells used, consist of sensory neurons including trigeminal neurons and dorsal root ganglion neurons. In more particular embodiments the primary cells consist of porcine trigeminal neurons.

In particular embodiments of the present invention the continuous cells used, consist of neuron-like cells such as for example a continuous cell line derived from trigeminal ganglion neurons, PC12 cells (CRL-1721) or ND7 cells; and non-neuronal cells such as for example swine testicle cells, swine kidney cells (e.g. PK15 (CCL-33), SK-RST(CRL-2842)), epithelial cell cultures, skin keratinocytes (e.g. HEK001 (CRL-2404), CCD1102 (CRL-2310)), Vero cells (CCL-81), human fetal lung fibroblasts (e.g. HFL1 (CCL-153)), human embryonic lung cells (e.g. HEL299 (CCL-137)) or lymphocyte cell cultures. In one object the continuous cell line used, is derived from trigeminal neurons, PC12 cells or ND7 cells. In more particular embodiments of the present invention the continuous cell line consist of an established cell line derived from porcine trigeminal neurons.

In the methods of the present invention the cells (permissive cells) are contacted with the cytokine till the cells are free of detectable viral proteins.

It is accordingly an object of the present invention to provide the methods to induce viral latency in a virus infected cell as described herein, said methods further comprising the step of;
- contacting said cells with interferon alpha till the cells are free of detectable viral proteins.

As described hereinbefore, the presence of infectious viral particles in the media can be determined using any one of the available protein measurement techniques and is typically determined using late viral specific antibodies, in particular using polyclonal anti-viral serum.

In either of the aforementioned methods to induce viral latency in a virus infected cell, the cells are optionally treated with interferon alpha prior to viral infection of said cells. In this alternative embodiment the cells are pretreated with the cytokine, in particular for at least 1 hour till 72 hours prior to viral infection of said cells; more in particular from 10 hours till 48 hours prior to viral infection of said cells; more in particular 24 hours prior to viral infection of said cells.

It is accordingly an object of the present invention to provide a method to induce viral latency in a alphaherpesvirus infected neuron-like cell, said method comprising;
- pretreating said cells with interferon alpha prior to viral infection of said cells;
- infect said cells with a virus;
- contacting said virus infected cells with interferon alpha ; and
characterized in that permissive cells are used and that no attenuated virus mutants, and/or exogenous (as in non-naturally occurring) antiviral compounds are used.

Viral infection of the cells, i.e. of the primary or continuous cells as defined above, is done using art known conditions and typically comprise supplying the cell medium with 0.01 to 1000 plaque forming units (PFU) per cell; in particular from 0.1 to 100 PFU per cell; more in particular using 1 to 10 PFU per cell.

The viruses used in the present invention can be obtained by purchasing them commercially (as part of a cell line or tissue sample) from for example ATCC or by obtaining them according to procedures well known in the art, such as by obtaining clinical isolates, or cultures from researchers in the field. Textbooks which discuss manipulations of viruses are many, including: Fields & Knipe, Fundamental Virology; Luria et al., General virology; and Fenner et al., Molecular Virology. Within the meaning of each of the viruses mentioned herein, the strains of each virus type are, of course, included within the scope of the present invention. For instance, human HSV-2 includes the HSV-2 strains G and 333.

In either of the different objectives of the present invention, the viruses as used herein are

Alphaherpesviruses, such as for example herpes simplex virus 1, herpes simplex virus 2, varicella zoster virus, varicellovirus, mardivirus, porcine alphaherpesvirus or iltovirus. In a particular embodiment the viruses are selected from the group consisting of herpes simplex virus 1 (HSV-1), herpes simplex virus 2 (HSV-2) and porcine alphaherpesvirus (PRV); more in particular HSV-1 or PRV.

In further embodiment, the cells in the aforementioned methods are treated either with interferon alpha, or with interferon alpha in combination with at least one 'further' cytokine selected from the group consisting of interferon beta, interferon gamma, interferon lambda, tumor necrosis factor alpha, tumor necrosis factor gamma, interleukin 1, interleukin 2, interleukin 4, interleukin 6, interleukin 8, interleukin 10, interleukin 12 and interleukin 18; in particular with interferon alpha or with interferon alpha in combination with a cytokine selected from interferon beta, tumor necrosis factor alpha or interleukin 10.

In analogy with the other methods of the present invention, in the aforementioned method the cells are contacted with either interferon alpha or interferon alpha in combination with at least one 'further' cytokine till;
- the media is free of detectable infectious viral particles; or
- at least 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% of the infected cells no longer show detectable viral protein expression.

Again, in an alternative embodiment of the aforementioned method the cells are treated with interferon alpha or interferon alpha in combination with at least one 'further' cytokine, prior to viral infection of said cells.

In a particular embodiment, the present invention provides a method to induce alphaherpesvirus latency in infected sensory neurons, said method comprising;
- contacting said sensory neurons with interferon alpha till either;
   o the medium is free of detectable infectious viral particles ; or
   o at least 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% of the infected cells no longer show detectable viral protein expression; and
characterized in that said sensory neurons are permissive for alphaherpesvirus and that no attenuated virus mutants, and/or exogenous (as in non-naturally occurring) antiviral compounds are used.

In an alternative embodiment the sensory neurons are treated with interferon alpha prior to viral infection, in particular using the conditions described hereinbefore. In an further embodiment the sensory neurons consist of trigeminal neurons, in particular porcine trigeminal neurons and the herpesvirus is selected from the group consisting of herpes simplex virus 1, herpes simplex virus 2 and porcine alphaherpesvirus; more in particular the virus consists of porcine alphaherpesvirus.

As already mentioned hereinbefore, the methods as provided in the different embodiments of the present invention are performed under physiologically relevant conditions. Said conditions are meant to refer to the normal (wild-type) physiologically conditions known for the viral strain and host used. Such as for example at an incubation temperature of about and between 35 - 40°C, i.e. using the art known and established cultivation conditions for the host cells used. In general, the reactivatable latent state can be achieved without the need of an elevated incubation temperature, and/or the use of attenuated viruses, and/or the use of exogenous antiviral compounds.

Reactivation of latently infected cells is done using art known procedures and include the use of condition selected from heat shock, forskoline treatment, UV treatment, corticosteroid (eg dexamethasone) or other (neuro)hormone treatment, nerve growth factor deprivation for neuronal cultures, histone deacetylase inhibitor treatment, axotomy and superinfection; in particular using forskoline treatment. Certain cytokines have also been demonstrated to be potent activators of latent virus. WO00/65355 for example discloses that IL-2 and TNF-alpha are potent activators of latent HIV virus. In WO99/16891 it was demonstrated that reactivation of latent HMCV is dependent on IFN-gamma production.

### Screening methods

In a further embodiment, the present invention provides methods to identify compounds capable of modulating the induction of viral latency in a neuron-like cell.

As described hereinbefore said methods comprise either the use of the methods to induce viral latency as provided above or of the cells obtainable using the methods of the present invention.

In a first aspect, i.e. using the methods to induce viral latency in a alphaherpesvirus infected neuron-like cell, the level of latency obtained in the presence of the compound to be tested is compared with the level of latency obtained in the absence of said compound.

The level of viral latency is determined using art known procedures, such as for example by determining the presence of viral protein in virally infected cells. Measurement of viral proteins uses art-known procedures as described hereinbefore.

In a particular embodiment the level of viral latency is determined by assessing the percentage of cells in which immediate early viral proteins can be detected. As shown in the examples hereinafter, using the methods of the present invention and in the absence of a compound to be tested, only up to 50% of the cells in which no detectable late viral protein expression can be determined, show detectable immediate early viral protein expression. A further reduction in the percentage of cells in which no detectable late viral protein expression can be determined is indicative for an increase of the fraction of latent cells in said population.

It is accordingly an object of the present invention to provide a method to identify a compound capable to modulate the induction of viral latency in a neuron-like cell, said method comprising;
- applying the methods according to the invention in the presence and absence of the compound to be tested; and
- compare the level of viral latency obtained in the presence and absence of the compound to be tested;
wherein a compound capable to reduce the percentage of cells in which immediate early viral proteins can be detected when compared to the percentage obtained in the absence of the compound to be tested, is identified as a compound that promotes viral latency in a cell.

In a particular embodiment said method comprises;
a) contacting an alphaherpes viral infected neuron-like cell with interferon alpha till either;
   a. the media is free of detectable infectious viral particles; or
   b. at least 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% of the infected cells no longer show detectable viral protein expression
b) contacting an alphaherpes viral infected cell with interferon alpha and the compound to be tested using the same conditions as in step a) above; and
c) compare the level of viral latency obtained in the presence (step b)) and absence (step a)) of the compound to be tested;
wherein a compound capable to change the level of viral latency when compared to the level obtained in the absence of said compound, is a compound capable of modulating the induction of viral latency in a cell.

The same conditions as mentioned in the method hereinbefore, in particular means that the incubation period for the cells in step b) is held identical to the incubation period determined in step a), irrespective of the presence of infectious viral particles in the media or of the percentage of infected cells that no longer show detectable late viral protein expression.

In a second objective, i.e using the cells obtainable using the methods to induce viral latency of the present invention, the method to identify a compound capable to modulate the reactivation from viral latency in a viral infected cell, comprises contacting the latent virally infected cells with the compound to be tested and determine whether said compound is capable to influence viral reactivation in said cells. A compound capable to prevent viral reactivation in said cells is identified as an anti-viral compound useful in the treatment of latent viral infections as described herein.

The cells used in these screening methods are the ones described hereinbefore and in particular consist of sensory neurons, more in particular of porcine trigeminal neurons.

In a particular embodiment of the aforementioned method, the cells are latently infected with alphaherpesviruses; in particular herpesviruses selected from the group consisting of herpes simplex virus 1, herpes simplex virus 2 and porcine alphaherpesvirus, i.e. pseudorabies virus; more in particular with porcine alphaherpesvirus, i.e. pseudorabies virus or herpes simplex virus 1.

This invention will be better understood by reference to the Experimental Details that follow, but those skilled in the art will readily appreciate that these are only illustrative of the invention as described more fully in the claims that follow thereafter. Additionally, throughout this application, various publications are cited, more fully the state of the art to which this invention pertains.

### EXAMPLES

The following examples illustrate the invention. Other embodiments will occur to the person skilled in the art in light of these examples.

### MATERIALS & METHODS

### Cultivation, virus inoculation, and analysis of primary TG neuronal cultures in a two-chamber model

Porcine trigeminal ganglion (TG) neurons were obtained as described before (Geenen et al., 2005) and seeded in an in vitro model, based on the 'Campenot' system, that allows to simulate the in vivo route of neuronal infection (De Regge et al, 2006ab). In brief, porcine trigeminal ganglia were excised from 4 to 6 week old piglets and dissociated by enzymatic digestion with 0.2% collagenase A (Roche, Mannheim, Germany). The harvested cells were resuspended in culture medium (MEM supplemented with 10% fetal bovine serum, 10OU/ml penicillin, 0,1 mg/ml streptomycin, 0,1 mg/ml kanamycin and 30 ng/ml nerve growth factor (Sigma Chemical Compagny, St.Louis, MO, USA)) and seeded in the inner chamber of an in vitro two-chamber model. The two-chamber system consists of a polyallomer tube that is fixed with silicon grease on a collagen coated cover glass inserted in a 6-well plate (Nalge Nunc International, Rochester, NY, USA). The inside of the tube forms the inner chamber, the outside forms the outer chamber. The silicon barrier prevents diffusion of medium or virus from one chamber to the other (De Regge et al, 2006a). One day after seeding, cultures were washed with RPMI (Gibco BRL, Life Technologies Inc., Gaithersburg, MD) to remove non-adherent cells and from then on, culture medium was changed three times a week. After two to three weeks of cultivation, when clear axon growth could be observed in the outer chamber, two chamber models were ready for inoculation with PRV or HSV-1.

Infections were done with wild type PRV strain Becker (Card et al, 1990) or wild type HSV-1 strain F(VR-733). Respectively 2 hours and 48 hours after inoculation of the outer chamber with 2×10⁷ PFU of PRV or HSV-1, medium containing PRV or HSV-1 was removed and the outer chamber was washed twice with culture medium. Afterwards new culture medium supplemented with polyclonal antibodies to PRV or HSV-1 and guinea pig complement (Sigma-Aldrich) was added to prevent further continuous infection pressure from the outer chamber to neurons in the inner chamber. In experiments where interferons were used, both the inner and outer chamber were pretreated with interferon for 24h. Except for the presence of interferon, the infection was done as in control experiments. After the pretreatment of 24h interferon was no longer present in the outer chamber but remained present in the inner chamber for the total time of the experiment as indicated in the text.

The ratio between viral antigen positive neurons in the inner chamber to the number of axons in the outer chamber after different treatments was determined by fluorescent labeling of neurons and viral antigens. For each condition, at least 20 axons with outgrowth in the outer chamber were examined. Data shown represent means ± SEM of triplicate assays.

### Antibodies, proteins and chemicals

Polyclonal porcine FITC-labeled anti-PRV antibodies were produced as described before (Nauwynck and Pensaert, 1995). Polyclonal rabbit anti-IE180 antibodies were a gift of Prof. Tabares (Gomez-Sebastian and Tabares, 2004). The monoclonal neuronal markers mouse anti-neurofilament 68 and rabbit anti-neurofilament 200 were purchased from Sigma-Aldrich and monoclonal mouse anti-HSV-1-gD antibodies were purchased from Santa Cruz Biotechnology Texas red- or FITC-labeled goat anti-mouse and goat anti-rabbit antibodies were obtained from Invitrogen. Recombinant porcine interferon alpha (PBL Biomedical Laboratories) and gamma (R&D systems) were used. Forskolin was obtained from Sigma-Aldrich.

### Immunofluorescence staining procedures

After being washed in PBS, neuronal cultures in the inner and outer chamber of the two-chamber model were fixed in 100% methanol for 20 min at -20°C. All antibodies were diluted in PBS, to a dilution of 1:100. Cells were incubated with each antibody for 1h at 37°C and were washed two times in PBS in between all incubations steps and after the last incubation step.

### Confocal microscopy

Stainings were analysed on a Leica TCS SP2 laser scanning spectrum confocal system (Leica Microsystems GmbH, Heidelberg, Germany) linked to a Leica DM IRBE microscope (Leica Microsystems GmbH). Images were taken using a 63x oil objective (NA 1.40-0.60) at room temperature and using Leica confocal acquisition software (Leica Microsystems GmbH). Adjustments of brightness and contrast were applied to the entire images and were done using Adobe Photoshop (Adobe Systems Inc., San Jose, CA).

### RESULTS

### Interferon alpha and gamma suppress pseudorabies virus replication in TG neurons at 24h post inoculation

Two-chamber models, consisting of TG neuronal cultures grown in an inner chamber with outgrowth of axons to an outer chamber separated from the inner chamber by a virus- and medium-impermeable silicon barrier (De Regge et al, 2006ab), were used to study the antiviral effect of IFN-α and -γ on a PRV infection of TG neurons. In a first step, a quantification method was established that allows to detect a possible suppressing activity of interferons on productive virus replication. To this end, PRV infected two-chamber models were simultaniously stained for neurofilament (to visualize neurons) and late viral antigens (to visualize productive virus replication). When two-chamber models were analysed at 24h post inoculation (pi) with 2.10⁷ plaque forming units (PFU) of PRV, the ratio between the number of viral antigen-positive neurons in the inner chamber to the number of axons grown through the silicon barrier into the outer compartment almost equaled one. This shows that for each axon that grows into the outer chamber, the corresponding neuron is productively infected in the inner chamber. This ratio can therefore be used to quantify the effect of interferon on productive viral replication in TG neurons.

The effect of several doses of IFN-α and -γ at 24hpi was analysed (Figure 1). In these experiments, the inner and outer chamber of the two-chamber model were pretreated with either of the IFN' s for 24h, followed by the addition of 2.10⁷ PFU of PRV to the outer chamber. At 24hpi in the presence of interferons, the two-chamber models were fixed and processed as described above. Quantification showed that both interferons reduced productive virus infection in a dose-dependent manner with a reduction ranging from 64% at 0.5 U/ml to 98% reduction at 500 U/ml for IFN-α. Reduction with IFN-γ was less pronounced, ranging from 45% at 0.5 ng/ml to 81% at 50 ng/ml.

These results show that interferons, especially IFN-α, are able to efficiently repress productive virus replication in TG neurons up to 24hpi.

### Interferon alpha suppresses pseudorabies virus replication in TG neurons for several days

To address if the suppressive effect of IFN-α is sustained over a longer period of time, two-chamber models were pretreated with 500U/ml IFN-α for 24h, followed by inoculation with PRV in the outer chamber and fixation at 120hpi. IFN-α was present in the inner chamber during the entire experiment. After staining and analysis of the two-chamber models, 3 different stages of infection were observed (Figure 2). The vast majority (± 90%) of TG neurons showed no detectable expression of late viral antigens, indicating that the virus was in a repressed state in these neurons and did not lead to productive virus infection. In a small percentage of neurons (± 6%), late viral antigen expression was limited to the golgi without spread of the virus to non-neuronal cells. In an even smaller percentage of cells (± 4%), infected neurons were found in which new infectious virus particles were formed that had spread to the non-neuronal cells surrounding the cell body. These results indicate that in a large majority of TG neurons, IFN-α is able to suppress late viral antigen expression to undetectable levels up to 120hpi. The same experiment was conducted in the presence of 50 ng/ml IFN-γ. In this condition, all two-chamber models showed extensive virus spread in the inner chamber at 120hpi, indicating that the virus had overcome the antiviral state of the cells induced by IFN-γ (data not shown).

These results indicate that IFN-α is able to efficiently suppress alphaherpesvirus replication for several days.

### Interferon alpha leads to a total viral protein shutdown in a majority of infected TG neurons at 120h post inoculation

Previous results showed that IFN-α had a strong suppressive effect on the expression of late viral proteins in PRV infected TG neurons. In a next experiment, the effect of IFN-α on the immediate early (IE) protein expression level was examined by performing immunofluorescent stainings with antibodies directed to the only IE protein of PRV, i.e. IE180, and neuronal antigens. IE180 is a transcription factor essential to initiate viral replication and its HSV-1 homologue ICP4 is localised in discrete nuclear foci early during infection (de Bruyn Kops, 1998; Everett et al, 2004). In control infection experiments, without the use of IFN-α, IE180 expression localised in specific compartments in the nucleus could only be detected in a minority of infected neurons for a brief period early in infection, between 6 and 8 hpi (data not shown). In the presence of 500U/ml IFN-α however, IE180 proteins localised in discrete nuclear compartments were detected in all infected neurons at 24hpi (Figure 3). At 120hpi in the presence of IFN-α however, localized nuclear IE180 expression was observed in only 45% of the infected neurons. The other 55% of infected neurons did not show any staining, indicating that these infected neurons had no detectable viral gene expression at that timepoint.

These data indicate that all infected TG neurons proceed to expression of IE180 protein in the presence of IFN-α. However, within 5 days post inoculation, IE180 expression is reduced to undetectable levels in de majority of infected TG neurons.

### Interferon alpha leads to a stable dormant state of infection in a majority of infected TG neurons at 120h post inoculation

Seen the strong suppressive effect of 500U/ml IFN-α, abolishment of late viral gene expression in 90% of the infected neurons and repression of IE180 expression to undetectable levels in 55% of the infected neurons after 120hpi, the fate of infected neurons after the withdrawal of IFN-α was examined. Two-chamber models were preatreated with 500U/ml IFN-α and infected with 2.10⁷ PFU of PRV. At 120hpi, IFN-α was removed from the inner chamber by washing the cells 2 times with MEM and new culture medium without IFN-α was added to the cells. Two-chamber models were then further cultured for 3 days and finally were fixed at 192hpi, followed by staining with antibodies to visualize neurons and late viral antigens. Quantification showed that after an incubation period of 3 days without the suppressing effect of IFN-α, 60% of the neurons still did not show detectable expression of late viral proteins (Figure 4), indicating that the virus is stably suppressed in these cells. When the same experiment was performed, and two-chamber models were stained for the presence of IE180 protein instead of late viral antigens, no infected neurons showing IE180 protein expression in discrete nuclear foci could be detected (data not shown).

These results indicate that at 5 days post inoculation of TG neurons in the presence of IFN-α, the majority of neurons contains the virus in a stable dormant state.

### Forskolin treatment causes reactivation of PRV from the dormant state of infection induced by interferon alpha

To determine whether the IFN-α induced dormant state represents a true latent infection, we examined if the virus is able to reactivate from this dormant state. Two-chamber models were infected and treated with IFN-α as described before. At 120 hpi, IFN-α was washed away an medium supplemented with forskolin was added to the neurons. Forskolin is a product that interferes with cAMP signaling in cells and is known to reactivate HSV-1 from quiescently infected primary neurons and neuronlike continuous cells in culture (Smith et al, 1992; Colgin et al, 2001; Danaher et al, 2003). Again, 3 days after replacement of the medium, two-chamber models were fixed, stained and analysed. Figure 5 shows that 3 days after forskolin treatment, 70% of the infected neurons showed expression of late viral proteins, compared to only 40% when medium without forskolin was added. This experiment shows that a forskolin treatment induces a reactivation of PRV in 50% of the neurons that contained dormant virus at 120hpi.

These reactivation data indicate that the IFN-α induced dormant state of the virus represents true viral latency.

### Interferon gamma can substitute for interferon alpha in maintaining PRV in a latent state of infection

We found earlier that IFN-γ suppresses viral replication in TG neurons at 24 hpi but , in contrast to IFN-α, is not able to suppress productive viral replication for several days. However, IFN-γ has been shown to be an important factor in maintaining alphaherpesviruses in a latent state of infection in vivo and in vitro. In this context, we examined whether upon initial virus suppression by IFN-α, IFN-γ was able to substitute for IFN-α to retain the virus in a repressed state. To this end, two-chamber models models were infected and treated with IFN-α as described above. At 120hpi, the medium containing IFN-α was replaced after several washes by medium supplemented with 50 ng/ml IFN-γ. Two-chamber models were fixed, stained and analysed at 192hpi. Over 90 % of the infected neurons did not show detectable late viral protein expression whereas 40% of the neurons proceeded to late viral protein expression when medium without IFN was added (Figure 6).

This shows that the number of infected neurons without late viral protein expression present after being incubated till 120hpi with medium containing IFN-α remains unchanged when the suppressing effect of IFN-α in the medium is replaced by the antiviral effect of IFN-γ.

### Herpes simplex virus 1 behaves similarly as pseudorabies virus in interferon alpha-treated porcine trigeminal ganglion neurons

To determine whether the latency-inducing effect of IFN-α in PRV-infected TG neurons, as is shown in previous results, may hold true for alphaherpesviruses in general, the effect of IFN-α on HSV-1 replication in porcine TG neurons was examined.

In analogy with the experiments performed with PRV, HSV-1 infected two-chamber models were simultaneously stained for neurofilament (to visualize neurons) and HSV-1-gD late antigen (to visualize productive virus replication). When two-chamber models were analysed at 48h post infection (pi) with 2.10⁷ plaque forming units (PFU) of HSV-1, the ratio between the number of viral antigen-positive neurons in the inner chamber to the number of axons grown through the silicon barrier into the outer compartment equaled 0.12 ± 0.01. This shows HSV-1 completes its replication cycle in 12 % of neurons that have been in contact with the virus.

In a next step, the effect of 500 U/ml IFN-α on HSV-1 replication was analysed at 48 and 120hpi (Figure 7). In these experiments, the inner and outer chamber of the two-chamber model were pretreated with 500 U/ml IFN-α for 24h, followed by the addition of 2.10⁷ PFU of HSV-1 to the outer chamber. At 48 and 120hpi in the presence of IFN-α in the inner chamber, two-chamber models were fixed and processed as described above. Quantification showed that 500U/ml of IFN-α completely abolished productive HSV-1 infection at both timepoints. These results show that IFN-α is able to efficiently repress productive HSV-1 replication in TG neurons up to 120hpi.

Furthermore, the stability of the suppressed state was examined by the withdrawal of IFN-α from the inner chamber at 120 hpi. At this timepoint, the inner chamber was washed twice with MEM and new culture medium without IFN-α was added to the cells. Two-chamber models were then further cultured for 3 days and fixed at 192hpi, followed by staining with antibodies to visualize neurons and late viral antigens. Quantification showed that after an incubation period of 3 days without the suppressing effect of IFN-α, no detectable expression of HSV-1 gD protein was found in any neuron (Figure 7), indicating that the virus is stably suppressed in the infected neurons.

These results indicate that at 5 days post inoculation of TG neurons in the presence of IFN-α, HSV-1 is present in a stable dormant state in all the infected neurons, and thus indicates that HSV-1 behaves in a similar way as PRV, and that the latency-inducing effect of IFN-α holds true for alphaherpesviruses in general.

### DISCUSSION

The ability of alphaherpesviruses to establish a latent infection in neurons of its host is a very important aspect
of the lifecycle of these viruses. It allows them to remain present in the host for the entire lifetime and to reactivate in response to several stimuli, associated with the production of new virus that can cause recurrent disease symptoms and can spread to new hosts. Many studies have been undertaken, both in vivo and in vitro, to unravel the mechanisms that lead to the induction and maintance of this latent state of infection and resulted in the commonly accepted view that the latency/reactivation cycle is controlled by a poorly understood interaction between virus, neurons and immune system (Divito et al, 2006). Many questions and doubts remain, especially since the latency/reactivaton cycle has up to date not been recapitulated using wild type virus, neurons and components of the immune system in vitro (Efstathiou & Preston, 2005). In this study, we used a homologous in vitro two-chamber model, based on the porcine alphaherpesvirus PRV and porcine TG neurons, that allows to mimic the natural route of infection to study the role of the immune system, more in particular of interferons, in the establishment of a latent infection. Using our model, we found that IFN-α and -γ were able to block virus replication at 24hpi at a point before 'late' viral protein expression, but IFN-α showed a stronger dose-dependent reduction in the number of late protein expressing neurons compared to IFN-γ. The suppressive effect of IFN-α was sustained for longer periods of time since 90% of the infected neurons still did not show detectable late viral gene expression at 120hpi. During a herpesvirus infection, viral proteins are expressed in a sequential fashion after the genome has been transported to the nucleus. Late viral proteins are expressed late in infection and are accompagnied by infectious virus production and virus spread. Immediate early proteins are the very first viral proteins expressed after infection and constitute transcription factors that initiate the subsequent transcription of early and late proteins. Without these immediate early proteins, viral replication does not initiate (Roizman & Knipe, 2001). PRV expresses only one protein with IE kinetics, IE180, the functional homologue of ICP4 of HSV-1 (Martin et al, 1990).

In untreated TG neurons, we found that, as expected, PRV IE180 was present in discrete nuclear foci early in infection (6-8hpi) in a minority of infected neurons. However, when our two-chamber model was infected in the presence of IFN-α, all infected neurons showed IE180 protein expression located in specific nuclear compartments at 24hpi. Because of the generally present, long lasting localised expression of IE180 in infected IFN-α treated neurons in comparison to the low abundant, short living localised expression in infected non-treated neurons, it is tempting to speculate that IFN-α blocks PRV replication after IE expression before the onset of early protein expression in TG neurons. This would confirm the results obtained in primary macrofages and continuous cell lines. Although the hypothesis that IFN-α blocks viral replication between immediate early and early protein expression needs further investigation, our current results already reveal crucial information about the state of the viral genome in the IFN-α treated infected neurons. They show that IFN-α has no gross influence on the entry of PRV in the neuronal axon endings and subsequent retrograde spread to the nucleus and proove that all infected neurons received a functional viral genome. Interestingly, at 120hpi in the presence of IFN-α, only 45% of the infected neurons showed detectable IE180 expression. This shows that 55% of the infected neurons do no longer show any detectable viral gene expression after a 5 days incubation period with IFN-α, and since latently infected neurons per definition do not show any viral gene expression (Roizman & Knipe, 2001) raises the possibility that the viral genome was directed in a latent state in these neurons.

After removal of medium containing IFN-α from the inner chamber at 120hpi and replacing it by medium without any suppressive component, 60% of the infected neurons did not proceed to detectable expression of late viral proteins within the next 3 days, indicating that the virus is stably suppressed in these cells. Of possible importance is the correlation between the number of neurons that do not express late viral antigens 3 days after withdrawal of IFN-α at 120hpi (60%) and the number of neurons that no longer showed detectable IE180 expression at 120hpi (55%). This leads to the hypothesis that specifically those neurons that still express IE180 at 120hpi proceed to expression of late viral proteins when the suppressive stimulus is withdrawn. This is supported by the observation that at 3 days after IFN-α was removed at 120hpi, no IE180 expression in distinct nuclear compartments could be detected in any neuron. This indicates that a total shut-down of viral protein expression is necessary to install a stably suppressed state of viral infection in neurons and that the presence of IE proteins suffices to proceed to productive infection upon removal of the suppressive agent.

An important aspect in the definition of a true latent infection is the possibility of the virus to reactivate from the latent state. Reactivation in vivo is mostly observed after physical or psychological stress or in immunocompromised patients and is correlated with a suppression of the immune status of the host. The exact molecular mechanism leading to reactivation is not yet known. Several stimuli have been shown to lead to reactivation of HSV-1 from latently infected mice in vivo and from quiescently infected cells in vitro (Smith et al, 1992; Colgin et al, 2001; Danaher et al, 2003; Hunsperger & Wilcox, 2003ab). One of these methods is the treatment of the cells with forskolin, a molecule interacting with cAMP signaling in the cell. We found that forskolin induced reactivation of PRV in our two-chamber model in 50% of neurons containing stably suppressed virus at 120hpi. This confirms that the stably suppressed virus infection in TG neurons can be reactivated and therefore represents true viral latency. Additional experiments with HSV-1 showed that the latency-inducing effect of IFN-α that we observed with PRV may also hold true for HSV-1 and alphaherpesviruses in general.

A latent herpesvirus infection is defined as the presence of a functional wild type viral genome in neurons of the host without the production of new virus particles. Upon specific stimuli, the virus can reactivate and produce new infectious virus particles that may spread and cause recurrent disease symptoms. The results discussed above show that PRV infection of TG neurons in our two-chamber model in the presence of IFN-α fullfills all criteria mentioned in the definition and represents the first induction of a latent state of infection in vitro making use of a wild type alphaherpesvirus, physiologically relevant culture conditions, and physiologically relevant, endogenous components of the immune system.

With this in vitro model in which a natural latent alphaherpesvirus infection can be induced, we provide a promising tool to dissect the molecular details of alphaherpesvirus latency and reactivation. Besides its usefullness in fundamental research, several possible application purposes are situated in the field of drug development. Till now, very little drugs are available to treat recurrent symptoms associated with reactivation of alphaherpesviruses, all of them being derivates of acyclovir (Woo & Challacombe, 2007). Our model could be a valuable tool to identify components that interfere with the latency/reactivation cycle, thereby being possible candidates for curative treatment of recurrent disease symptoms. For about 20 years, HSV has been studied as a promising vector to deliver transgenes to neurons, in this way helping to cure neuronal diseases (Broberg & Hukkanen, 2005). Our model could be a helpfull tool to screen the capacity of recombinant alphaherpesviruses designed for neuronal gene therapy to go into a latent state of infection under physiological relevant conditions and their capacity for high and long lasting expression of the transgene.

In conclusion, we show that addition of IFN-α to neurons of the trigeminal ganglion in vitro is sufficient to drive wild type PRV in a stable but reactivatable latent state of infection in a majority of infected TG neurons. This is the first physiologically relevant recapitulation of the latency/reactivation cycle of alphaherpesviruses.

### References

Broberg, E. K., and V. Hukkanen. 2005. Immune response to herpes simplex virus and gamma 134.5 deleted HSV vectors. Cuff Gene Ther 5:523-30.
Card, J. P., L. Rinaman, J. S. Schwaber, R. R. Miselis, M. E. Whealy, A. K. Robbins, and L. W. Enquist. 1990. Neurotropic properties of pseudorabies virus: uptake and transneuronal passage in the rat central nervous system. J Neurosci 10:1974-94.
Colgin, M. A., R. L. Smith, and C. L. Wilcox. 2001. Inducible cyclic AMP early repressor produces reactivation of latent herpes simplex virus type 1 in neurons in vitro. J Virol 75:2912-20.
Danaher, R. J., R. J. Jacob, and C. S. Miller. 2003. Herpesvirus quiescence in neuronal cells. V: forskolin-responsiveness of the herpes simplex virus type 1 alpha0 promoter and contribution of the putative cAMP response element. J Neurovirol 9:489-97.
de Bruyn Kops, A., S. L. Uprichard, M. Chen, and D. M. Knipe. 1998. Comparison of the intranuclear distributions of herpes simplex virus proteins involved in various viral functions. Virology 252:162-78.
De Regge, N., H. W. Favoreel, K. Geenen, and H. J. Nauwynck. 2006a. A homologous in vitro model to study interactions between alphaherpesviruses and trigeminal ganglion neurons. Vet Microbiol 113:251-5.
De Regge, N., H. J. Nauwynck, K. Geenen, C. Kmmmenacher, G. H. Cohen, R. J. Eisenberg, T. C. Mettenleiter, and H. W. Favoreel. 2006b. Alpha-herpesvirus glycoprotein D interaction with sensory neurons triggers formation of varicosities that serve as virus exit sites. J Cell Biol 174:267-75.
Doerig C, Pizer LI, Wilcox CL. 1991 Detection of the latency-associated transcript in neuronal cultures during the latent infection with herpes simplex virus type 1. Virology. Jul;183(1):423-6.
Efstathiou, S., and C. M. Preston. 2005. Towards an understanding of the molecular basis of herpes simplex virus latency. Virus Res 111:108-19.
Everett, R. D. 2000. ICPO induces the accumulation of colocalizing conjugated ubiquitin. J Virol 74:9994-10005.
Everett, R. D., G. Sourvinos, C. Leiper, J. B. Clements, and A. Orr. 2004. Formation of nuclear foci of the herpes simplex virus type 1 regulatory protein ICP4 at early times of infection: localization, dynamics, recruitment of ICP27, and evidence for the de novo induction of ND 10-like complexes. J Virol 78:1903-17.
Geenen, K., H. W. Favoreel, L. Olsen, L. W. Enquist, and H. J. Nauwynck. 2005. The pseudorabies virus US3 protein kinase possesses anti-apoptotic activity that protects cells from apoptosis during infection and after treatment with sorbitol or staurosporine. Virology 331:144-50.
Gomez-Sebastian, S., and E. Tabares. 2004. Negative regulation of herpes simplex virus type 1 ICP4 promoter by IE180 protein of pseudorabies virus. J Gen Virol 85:2125-30.
Halford WP, Gebhardt BM, Carr DJ. 1996 Persistent cytokine expression in trigeminal ganglion latently infected with herpes simplex virus type 1. J Immunol. Oct 15;157(8):3542-9.
Hunsperger, E. A., and C. L. Wilcox. 2003a. Capsaicin-induced reactivation of latent herpes simplex virus type 1 in sensory neurons in culture. J Gen Virol 84:1071-8.
Hunsperger, E. A., and C. L. Wilcox. 2003b. Caspase-3-dependent reactivation of latent herpes simplex virus type 1 in sensory neuronal cultures. J Neurovirol 9:390-8.
Martin, K. J., J. W. Lillie, and M. R. Green. 1990. Transcriptional activation by the pseudorabies virus immediate early protein. Genes Dev 4:2376-82.
Nauwynck, H. J., and M. B. Pensaert. 1995. Effect of specific antibodies on the cell-associated spread of pseudorabies virus in monolayers of different cell types. Arch Virol 140:1137-46.
Roizman, B., and D.M. Knipe. 2001. Herpes simplex viruses and their replication. 2399-2459. In: Knipe, D., and P.M. Howley (Eds). Fields Virology, 4 ed. Lippencott, Williams and Wilkins. Philadelphia, PA, USA.
Smith, R. L., L. I. Pizer, E. M. Johnson, Jr., and C. L. Wilcox. 1992. Activation of second-messenger pathways reactivates latent herpes simplex virus in neuronal cultures. Virology 188:311-8.
Smith RL, Escudero JM, Wilcox CL. 1994 Regulation of the herpes simplex virus latency-associated transcripts during establishment of latency in sensory neurons in vitro. Virology. Jul;202(1):49-60.
Wilcox C.L. and Johnson Jr. E.M. 1988. Characterization of nerve factor-dependent herpes simplex virus latency in neurons in vitro J. Virol 62:393-399
Wilcox CL, Crnic LS, Pizer LI. 1992 Replication, latent infection, and reactivation in neuronal culture with a herpes simplex virus thymidine kinase-negative mutant. Virology. Mar;187(1):348-52.
Woo, S. B., and S. J. Challacombe. 2007. Management of recurrent oral herpes simplex

## Claims

1. An in vitro method of inducing latency in alphaherpes-virus infected neuron-like cells, said method comprising contacting said cells with interferon alpha and **characterized in that** permissive cells are used and no attenuated virus mutants, and/or exogenous antiviral compounds are used.

2. The method according to claim 1 wherein the cells are selected from the group consisting of sensory neurons, sympathetic neurons and leukocytes.

3. The method according to any one of claims 1 to 2, further **characterized in that** the cells are treated with interferon alpha prior to viral infection of said cells.

4. The method according to any one of claims 1 to 3 further **characterized in that** the interferon alpha is administered till at least 40 % of the infected cells no longer show detectable viral protein expression.

5. The method according to claim 4 wherein;
- the cells are porcine trigeminal neurons;
- treated with interferon alpha prior to viral infection;
- the virus consists of the porcine alphaherpesvirus (PRV) or herpes simplex virus 1 (HSV-1); and
- the viral protein expression is determined using polyclonal anti-PRV or anti-HSV-1 serum.

6. A method to identify compounds capable of modulating the induction of viral latency in a neuron-like cell said method comprising;
- applying the methods according to any one of claims 1 to 5 in the presence and absence of the compound to be tested; and
- compare the level of viral latency obtained in the presence and absence of the compound to be tested; and
wherein a compound capable to change the level of viral latency when compared to the level obtained in the absence of the compound to be tested is a compound capable of modulating the induction of viral latency in a cell.

7. A method according to claim 6 wherein the level of viral latency is determined by assessing
(i) the percentage of cells in with no detectable expression of immediate early viral proteins; and
(ii) assessing within the percentage of cells of step (i) if the capability of viral reactivation is retained.

## Patentansprüche

1. Eine in-vitro-Methode zur Herstellung von Latenz in Neuronenähnlichen Zellen, die von einem Alpha-Herpesvirus infiziert worden sind, wobei im Rahmen besagter Methode die besagten Zellen mit Interferon alpha in Berührung gebracht werden und wobei die besagte Methode weiter dadurch charakterisiert ist, dass permissive Zellen zur Anwendung gelangen und dass keine attenuierten Virusmutationen und/oder exogenen antiviralen Wirkstoffe Verwendung finden.

2. Die Methode entsprechend Anspruch 1, wobei die Zellen aus einer Gruppe auszuwählen sind, welche die folgenden Mitglieder umfasst: sensorische Neuronen, sympathische Neuronen und Leukozyten.

3. Die Methode entsprechend einem der Ansprüche 1 oder 2, weiter dadurch charakterisiert, dass die Zellen vor der viralen Infektion der besagten Zellen mit Interferon alpha behandelt werden.

4. Die Methode entsprechend einem der Ansprüche 1 bis 3, weiter dadurch charakterisiert, dass das Interferon alpha so lange verabreicht wird, bis in mindestens 40 % der infizierten Zellen keine viralen Proteinexpressionen mehr nachweisbar sind.

5. Die Methode entsprechend Anspruch 4, wobei:
- es sich bei den Zellen um porzine Trigeminalneuronen handelt;
- die Behandlung mit Interferon alpha vor der viralen Infektion erfolgt;
- der Virus aus dem porzinen Alphaherpesvirus (PRV) oder dem Herpes-Simplex-Virus 1 (HSV-1) besteht; und
- die virale Proteinexpression mit Hilfe von polyklonalem Anti-PRV bzw. Anti-HSV-1-Serum ermittelt wird.

6. Eine Methode zur Identifizierung von zusammengesetzten Wirkstoffen, die eine Anpassung der Herstellung viraler Latenz in neuronenähnlichen Zellen gestatten, wobei die besagte Methode die folgenden Elemente umfasst:
- die Anwendung der Methoden gemäß einem der Ansprüche 1 bis 5 in Anwesenheit und Abwesenheit des zu prüfenden zusammengesetzten Wirkstoffs; und
- einen Vergleich zwischen dem Grad viraler Latenz in Anwesenheit bzw. Abwesenheit des zu prüfenden zusammengesetzten Wirkstoffs; und
wobei der zusammengesetzte Wirkstoff, der - auf der Grundlage des Vergleichs zwischen dem Grad viraler Latenz in Anwesenheit bzw. Abwesenheit des besagten zusammengesetzten Wirkstoffs - zur Veränderung des Grades viraler Latenz imstande ist, ein zusammengesetzter Wirkstoff ist, der die Anpassung der Herstellung viraler Latenz in einer Zelle zu bewirken vermag.

7. Eine Methode entsprechend Anspruch 6, wobei der Grad viraler Latenz wie folgt ermittelt wird:
(i) durch Beurteilung des prozentualen Anteils von Zellen ohne nachweisbare Expression sofort-früher bzw. "immediate early" viraler Proteine; und
(ii) durch Beurteilung innerhalb der bei Schritt (i) ermittelten Untergruppe, inwieweit die Fähigkeit zur viralen Reaktivierung beibehalten worden ist.

## Revendications

1. Une méthode in vitro induisant à la latence dans les cellules d'aspect neuronal infectées de l'alpha herpès virus, ladite méthode comprenant lesdites cellules de contact avec de l'interféron alpha et **caractérisée par le fait que** l'on utilise les cellules permissives et pas une version mutante atténuée du virus, et/ou l'on utilise des composés antiviraux exogènes.

2. La méthode conformément à la revendication 1 où les cellules sont sélectionnées dans le groupe qui consiste en des neurones sensoriels, des neurones sympathiques et des leucocytes.

3. La méthode selon une des revendications 1 à 2, caractérisée plus loin par le fait que les cellules sont traitées avec de l'interféron alpha avant l'infection virale desdites cellules.

4. La méthode selon l'une des revendications 1 à 3 caractérisée plus loin par le fait que l'interféron alpha est administré jusqu'à ce qu'au moins 40 % des cellules infectées ne montrent plus d'expression de protéines virales détectable.

5. La méthode selon la revendication 4 où;
- les cellules sont des neurones trijumeaux de porc;
- elles sont traitées avec de l'interféron alpha avant l'infection virale;
- le virus consiste en en alpha herpès virus porcin (HVP) ou un virus de l'herpès simplex 1 (VHS-1); et
- l'expression de la protéine virale est déterminée par l'utilisation de sérum polyclonal anti-PRV ou anti anti-VHS-1.

6. Une méthode d'identification des composés capable de moduler l'induction de la latence virale dans une cellule d'aspect neuronal comprise dans ladite méthode;
- l'application des méthodes conformément aux revendications 1 à 5 en présence et en l'absence des composés à mettre à l'essai; et
- comparer le niveau de la latence virale obtenu en présence et en l'absence des composés à mettre à l'essai; et
où un composé capable de changer le niveau de la latence virale une fois qu'il est comparé au niveau obtenu en l'absence du composé à mettre à l'essai est un composé capable de moduler l'induction de la latence virale dans une cellule.

7. Une méthode conformément à la revendication 6 où le niveau de latence virale est déterminé en évaluant
(i) le pourcentage de cellules dans lesquelles il n'existe pas d'expression détectable de protéine virale précoce immédiate; et
(ii) l'évaluation dans le cadre du pourcentage des cellules de l'étape (i) si la capacité virale de réactivation est conservée.
